(19) Europäisches Patentamt
European Patent Office
Office européen des brevets

(11) Veröffentlichungsnummer: **0 427 150 B1**

(12) **EUROPÄISCHE PATENTSCHRIFT**

(45) Veröffentlichungstag der Patentschrift: **06.09.95**

(51) Int. Cl.$^{6}$: **C12P 7/18**, C12P 7/58

(21) Anmeldenummer: **90121064.1**

(22) Anmeldetag: **02.11.90**

(54) **Verfahren zur Gewinnung von Sorbit und Gluconsäure bzw. Gluconat und dafür geeignete Zellmassen.**

(30) Priorität: **04.11.89 DE 3936757**

(43) Veröffentlichungstag der Anmeldung:
**15.05.91 Patentblatt 91/20**

(45) Bekanntmachung des Hinweises auf die Patenterteilung:
**06.09.95 Patentblatt 95/36**

(84) Benannte Vertragsstaaten:
**AT BE DE FR NL**

(56) Entgegenhaltungen:
**EP-A- 0 322 723**
**US-A- 4 755 467**

**ANALYTICAL BIOCHEMISTRY vol. 120, 1982, NEW YORK US Seiten 211 - 234; HANSRUEDI FELIX: "Permeabilized cells"**

**APPLIED MICROBIOLOGY AND BIOTECHNOLOGY vol. 29, 1988, U.H. CHUN et al.; BERLIN Seiten 19 - 24;**

**CHUN U.H. et al: "The simultaneous production of sorbitol from fructose andgluconic acid from glucose using an oxidoreductase of Z.mobilis"**

**APPLIED MICROBIOLOGY AND BIOTEHNOLOGY vol. 20, 1984, BERLIN Seiten 118 - 123;VIIKARI I.: "Formation of sorbitol by Z.mobilis"**

(73) Patentinhaber: **FORSCHUNGSZENTRUM JÜLICH GMBH**
**Wilhelm-Johnen-Strasse**
**D-52425 Jülich (DE)**

(72) Erfinder: **Rehr, Bert, Dr.**
**Kopernikusstrasse 66**
**W-5170 Jülich (DE)**
Erfinder: **Sahm, Hermann, Prof.**
**Wendelinusstrasse 71**
**W-5170 Jülich (DE)**

**EP 0 427 150 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf ein Verfahren zur Gewinnung von Sorbit und Gluconsäure bzw. Gluconat ausgehend von wäßrigen Glucose/Fructosemischungen durch Stoffumwandlung in Gegenwart von permeabilisierten Zellen von Zymomonas mobilis und sie umfaßt für das Verfahren brauchbare Zellmassen.

Es ist bekannt, daß in wäßrigen Glucose/Fructosemischungen enzymatisch mit Hilfe von Glucose-Dehydrogenase und Sorbit-Dehydrogenase bei gleichzeitiger Anwesenheit von Cofaktoren Sorbit und Gluconsäure bzw. Gluconat entstehen. Dieses Verfahren setzt die fortlaufende Zugabe oder Regenerierung der Cofaktoren voraus.

Es wurde daher bereits ein Verfahren zur mikrobiellen Umwandlung von Glucose und Fructose in wäßriger Lösung mit Hilfe von Glucose/Fructose-Transhydrogenase enthaltenden Bakterien und insbesondere von Zymomonas mobilis entwickelt (US-PS 4 755 467), bei dem entsalzte zellfreie Extrakte oder immobilisierte Zellen oder mit phosphatfreiem Puffer gewaschene, nichtwachsende Zellen verwendet werden, die ggf. permeabel gemacht sind, wofür im konkreten Beispiel eine Toloul-Behandlung in 10 %iger (v/v) Toluollösung diente. Alternativ sollen Gluconatkinase-negative Mutanten gebildet und angewandt werden.

Durch eine solche Toluolbehandlung, die auch von U.H.Chun u. P.L.Rogers in Appl. Microbiol. & Biotechnol. 29 (1988) 19-24 beschrieben wird, werden zwar permeabilisierte Zellen erhalten, die für die gewünschte Fermentation sehr nützlich sind, jedoch erscheint der Einsatz so erheblicher Toluolmengen für die Permeabilisierung nicht optimal und die Gefahr erheblich, daß im Zellmaterial Toluolreste verbleiben.

Ziel der Erfindung ist daher ein Verfahren zur Stoffumwandlung mit Hilfe von permeabilisierten Zellen, die mit geringeren Fremdsubstanzmengen reproduzierbar erhalten und einfach von verbleibenden Resten befreit werden können.

Das zu diesem Zweck entwickelte erfindungsgemäße Verfahren ist dadurch gekennzeichnet, daß man durch Behandlung mit einer zumindest 0,1%igen Kationtensidlösung permeabilisierte Zellen verwendet.

Als Kationtensid wird dabei insbesondere eine längerkettige quaternäre Alkylammoniumverbindung verwendet, wie sie insbesondere der Formel I

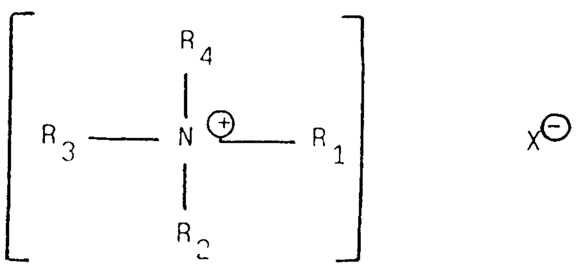

(I)

entspricht, in der zumindest einer der Reste $R_1$ bis $R_4$ ein Kohlenwasserstoffrest mit $\geqq$ 8 C-Atomen ist, während die übrigen Reste R gleich oder verschieden sein können und für Niederalkyl, insbesondere für $CH_3$- oder ggf. $C_6H_5$-$CH_2$- stehen und X ein verträgliches Anion, insbesondere Chlorid oder Bromid bedeutet.

Besonders zweckmäßig sind der Formel I entsprechende Kationtenside, bei denen $R_1$ und ggf. $R_2$ ein Alkylrest mit 10 bis 16 C-Atomen ist und die übrigen Reste R $CH_3$-Gruppen bedeuten, von denen eine einen Phenylrest tragen kann und X für Chlorid oder Bromid steht.

Dodigen® (Dodecyldimethylbenzylammoniumchlorid), Bardac® (Didecyldimethylammoniumchlorid) sowie Cetyltrimethylammoniumbromid (CTAB) und -chlorid gehören zu dieser Gruppe und sind im Handel erhältliche Kationtenside, die daher umfänglicher untersucht wurden, insbesondere das Cetyltrimethylammoniumbromid (CTAB).

Die Verwendung von Detergentien zur Permeabilisierung von Zellen allgemein ist zwar bereits seit längerer Zeit bekannt, wie aus dem Übersichtsartikel von H. Felix (Analytical Biochemistry 120, (1982), 211-23) hervorgeht, in dem auch das CTAB aufgeführt ist, jedoch zeigt die Übersicht, daß bislang vornehmlich nicht-ionische Detergentien eingesetzt wurden.

Es hat sich nun gezeigt, daß für die Behandlung von Zymomonas mobilis gerade Kationtenside in besonders geringer Menge angewandt werden können und zu einer optimalen Permeabilisierung führen.

Dies geht aus Vergleichsversuchen hervor, die mit verschiedenen Detergentien durchgeführt wurden:

Zu diesem Zweck wurden frisch angezogene Zellen in 0,1 M Natriumcitratpuffer (pH 6,4) in Mengen von 20 bis 30 g/l (Trockengewicht; Endkonzentration; allgemein sind Mengen bis zu 80 g/l zweckmäßig)

suspendiert und die Zellsuspension mit Detergenslösung für eine Endkonzentration von 0,1 % (CTAB, Dodigen und Bardac) bzw. 1 % (übrige Tenside) bzw. 10 % (Toluol) gemischt. Nach 10 minütiger Inkubationsdauer wurden die Zellen abzentrifugiert und durch zweimaliges Waschen mit Natriumcitratpuffer von Tensid- bzw. Toluolresten befreit.

Die so permeabilisierten Zellen wurden für die Umwandlung von wäßrigen Glucose/Fructosemischungen verwendet, und als Kontrolle für den Erfolg der Permeabilisierung durch Bestimmung eines Markerenzyms wurde die Konzentration von Glucose-6-phosphat-Dehydrogenase (Glucose-6-P-DH) gemessen (Glucose-6-P-DH ist ein intrazelluläres Enzym, dessen Aktivität nur bei aufgeschlossenen oder permeabilisierten Zellen gemessen werden kann, da die Zellmembran intakter Zellen für das im Messansatz vorhandene NADH impermeabel ist).

Die in der nachfolgenden Tabelle 1 angegebenen Aktivitäten sind Mittelwerte aus jeweils 7 Messungen mit zwei verschiedenen Zellernten. Der Null-Wert wurde mit unbehandeltem Zellmaterial erhalten. Als Rohextrakt wird durch Ultraschall aufgeschlossenes Zellmaterial bezeichnet.

Tabelle 1

| Permeabilisierung von Z. mobilis mit Toluol und verschiedenen Detergenzien | | | | |
|---|---|---|---|---|
| Behandlung mit | Konz. % | µmol Sorbit/mg Protein x min | µmol Gluconsr./mg Protein x min | Glucose-6-P-DH in µmol NADH/m Protein x min |
| O-Wert | | 0,2 | 0,2 | 0,25 |
| Rohextrakt | | 0,28 | 0,28 | 2,3 |
| CTAB | 0,1 | 0,23 | 0,2 | 2,0 |
| *Dodigen® 1611 | 0,1 | 0,18 | 0,18 | 2,0 |
| **Bardac® 22 | 0,1 | 0,21 | 0,21 | 1,9 |
| Tween® 80 | 1 | 0,18 | 0,2 | 0,8 |
| Brij® 58 | 1 | 0,2 | 0,2 | 0,5 |
| Nonidet®-P 40 | 1 | 0,22 | 0,22 | 0,9 |
| Lubrol® Px | 1 | 0,23 | 0,22 | 1,2 |
| Toluol | 10 | 0,23 | 0,22 | 1,8 |

* Dodecyldimethylbenzylammoniumchlorid
** Didecyldimethylammoniumchlorid

Aus diesen Daten folgt, daß eine optimale Permeabilisierung mit Hilfe von quaternären Ammoniumsalzen erreicht wird, was insbesondere aus der letzten Spalte ersichtlich ist, die zeigt, daß der Meßwert für das Markerenzym bei mit CTAB, Dodigen® oder Bardac® behandelten Zellen in der Größenordnung von demjenigen des Roh extrakts liegt, während der Enzymwert bei den anderen Detergentien deutlich niedriger ausfällt. Der höhere Meßwert für das Markerenzym gibt über eine stärkere Durchlässigkeit der Zellmembran Auskunft. Je durchlässiger nun die Zellmembran ist, umso rascher und vollständiger können die für den Zellstoffwechsel wichtigen Cofaktoren (NAD, ATP etc.) die Zelle verlassen, so daß eine Vergärung von Glucose und Fructose zu Ethanol und $CO_2$ sowie ein Abbau der Gluconsäure nicht mehr möglich ist, wodurch langzeitig eine maximale Produktausbeute erreicht wird. Aus der umfassenden Permeabilisierung resultiert also eine Produktausbeute, die nicht durch Ethanolbildung gemindert ist.

Für die Permeabilisierungsbehandlung werden insbesondere Tensid-Konzentrationen von 0,1 bis 0,3 % vorgesehen und die Behandlungsdauer liegt insbesondere bei 1 bis 10 Minuten bei Zimmertemperatur, wie aus den nachfolgenden Optimierungsversuchen hervorgeht:

A) Variation der CTAB-Konzentration

Die weiter oben für die Vergleichsversuche angegebene Verfahrensweise wurde wiederholt, wobei unterschiedliche CTAB-Konzentrationen angewandt wurden. Dabei wurden folgende Ergebnisse erzielt:

Tabelle 2

Permeabilisierung von Z. mobilis mit verschiedenen Konzentrationen von CTAB. Die Inkubationsdauer (Behandlung mit Detergenz) betrug 10 Minuten

| CTAB (%) | Glucose-6-P-DH in µmol NADH/mg Protein x min |
|---|---|
| 0 | 0,15 |
| 0,05 | 0,26 |
| 0,1 | 1,75 |
| 0,2 | 1,8 |

B) Variation der Behandlungszeiten

Analoge Versuche wurden unter Variation der Behandlungszeit durchgeführt. Die dabei erzielten Ergebnisse sind in der nachfolgenden Tabelle 3 zusammengefaßt.

Tabelle 3

Einfluß der Inkubationsdauer auf die Permeabilisierung von Z. mobilis mit CTAB. Die CTAB-Konzentration betrug 0,1 %

| Inkubationsdauer | Glucose-6-P-DH in µmol NADH/mg Protein x min |
|---|---|
| ohne CTAB | 0,12 |
| 1 min | 1,8 |
| 2,5 min | 1,68 |
| 5 min | 1,72 |
| 10 min | 1,75 |

Aus den vorstehenden Tabellen folgt, daß Konzentrationen unter 0,1 % CTAB nicht ausreichen um zu einer angemessenen Permeabilisierung zu kommen. Durch eine Steigerung der Konzentration über 0,3 % hinaus wird keine weitere Verbesserung erhalten. Die Permeabilisierung wird bereits innerhalb von einer Minute erreicht, jedoch erscheint es zweckmäßig, eine etwas längere Behandlungsdauer von bis zu 10 Minuten anzuwenden, um bei größeren Ansätzen lokale Schwankungen durch unzureichende Durchmischungen zu vermeiden.

Es folgen Beispiele für die Herstellung von Sorbit und Gluconsäure mit Hilfe der erfindungsgemäß permeabilisierten Zellen.

Umsetzung von Glucose und Fructose zu Sorbit und Gluconsäure im Kleinfermenter bei verschiedenen Zuckerkonzentrationen mit intakten und CTAB-permeabilisierten Zellen

die Umsetzung erfolgte bei 39 °C unter pH-statischen Bedingungen (0,1 M Natriumcitratpuffer pH 6,4; titriert mit 3 N NaOH)

A. Umsetzung mit intakten Zellen, Substratkonzentration je 123 g/l Fructose und Glucose, Zellkonzentration 22,1 g Protein/l

| - Zeit für die Umsetzung: 3 Stunden | |
|---|---|
| Sorbit: | 107 g/l (Effizienz der Umsetzung: 85,9 %) |
| Gluconsäure: | 77 g/l (Effizienz der Umsetzung: 57,5 %) |
| Fructose: | 30 g/l |
| Glucose: | 0 g/l |
| produziertes Ethanol: | 21 g/l |

B. Umsetzung mit CTAB-permeabilisierten Zellen, Substratkonzentration je 105 g/l Fuctose und Glucose, Zellkonzentration 14,4 g Protein/l

| - Zeit für die Umsetzung: 9,5 Stunden | |
|---|---|
| Sorbit: | 103 g/l (Effizienz der Umsetzung : 97 %) |
| Gluconsäure: | 110 g/l (Effizienz der Umsetzung : 99 %) |
| Glucose und Fructose: | 0 g/l |
| produziertes Ethanol: | ≦ 0,8 g/l |

C. Umsetzung mit CTAB-permeabilisierten Zellen, Substratkonzentration 200 g/l Fructose und 194 g/l Glucose, Zellkonzentration 23,3 g Protein/l

| - Zeit für die Umsetzung: 6 Stunden | |
|---|---|
| Sorbit: | 200 g/l (Effizienz der Umsetzung : 99 %) |
| Gluconsäure: | 209 g/l (Effizienz der Umsetzung : 99 %) |
| Glucose und Fructose: | 0 g/l |
| produziertes Ethanol: | ≦ 0,8 g/l |

D. Umsetzung mit CTAB-permeabilisierten Zellen, Substratkonzentration 292 g/l Fructose und 281 g/l Glucose, Zellkonzentration 39,6 g Protein/l

| - Zeit für Umsetzung: 6 Stunden | |
|---|---|
| Sorbit: | 285 g/l (Effizienz der Umsetzung : 97 %) |
| Gluconsäure: | 295 g/l (Effizienz der Umsetzung :96,5%) |
| Glucose und Fructose: | 0 g/l |
| produziertes Ethanol: | ≦ 0,8 g/l |

Die erhaltenen Ergebnisse sind in der nachfolgenden Tabelle 4 zusammengefaßt:

Tabelle 4

Vergleichende kinetische Daten ausgedrückt als maximale Produktivitäten in g Produkt/Stunde x g Protein

| Zellmaterial | $\frac{g\ Sorbit}{h \times g}$ | Ausbeute (%) | $\frac{g\ Gluconsäure}{h \times g}$ | Ausbeute (%) |
|---|---|---|---|---|
| intakte Zellen | 2,2 | 85,9 | 2,1 | 57,5 |
| CTAB-Zellen, siehe unter B. | 3,2 | 97 | 3,3 | 99 |
| CTAB-Zellen, siehe unter C. | 3,7 | 99 | 3,8 | 99 |
| CTAB-Zellen, siehe unter D. | 3,4 | 97 | 3,4 | 96,5 |

Wie man sieht, wird durch die Permeabilisierung mit CTAB die Ausbeute an Produkt gesteigert und es werden maximale Produktivitäten (die Umsatzgeschwindigkeit) erreicht. Die Ethanolbildung ist bei Verwendung permeabilisierter Zellen minimiert, so daß keine Ausbeuteverluste an gewünschtem Produkt durch eine solche Nebenproduktbildung zu verzeichnen sind.

Für die Stoffumwandlung werden insbesondere Zellkonzentration permeabilisierter Zellen von 20 - 80 g/l Zelltrockenmasse (entsprechend 10 - 40 g/l Protein) verwendet.

Die permeabilisierten Zellen können bevorratet und in den Handel gebracht werden.

Die permeabilisierten Zellen können in immobilisierter Form verwendet werden, was eine kontinuierliche Prozeßführung erlaubt. Dabei kann das Zellmaterial adsorptiv oder kovalent an einen anorganischen oder organischen Träger angelagert sein, der in feinteiliger oder stückiger Form oder als Formkörper vorliegen kann. Die Zellen können auch eingeschlossen in eine poröse Matrix verwendet werden.

Eine Rückhaltung der Zellen in einem Reaktor mittels feinporiger Membranen ist ebenfalls möglich, oder auch eine Abtrennung (z. B. durch Mikrofiltration) aus dem Reaktorablauf mit Rückführung der abgetrennten Zellen in den Reaktor.

## Patentansprüche

1. Verfahren zur Gewinnung von Sorbit und Gluconsäure bzw. Gluconat ausgehend von wäßrigen Glucose/Fructosemischungen durch Stoffumwandlung in Gegenwart von permeabilisierten Zellen von Zymomonas mobilis,
   **dadurch gekennzeichnet,**
   daß man durch Behandlung mit einer zumindest 0,1%igen Kationtensidlösung permeabilisierte Zellen verwendet.

2. Verfahren nach Anspruch 1,
   **dadurch gekennzeichnet,**
   daß man permeabilisierte Zellen verwendet, die durch Behandlung mit einer längerkettigen quaternären Alkylammoniumverbindung erhalten worden sind.

3. Verfahren nach Anspruch 2,
   **dadurch gekennzeichnet,**
   daß man permeabilisierte Zellen verwendet, die durch Behandlung mit einer Verbindung der Formel (I)

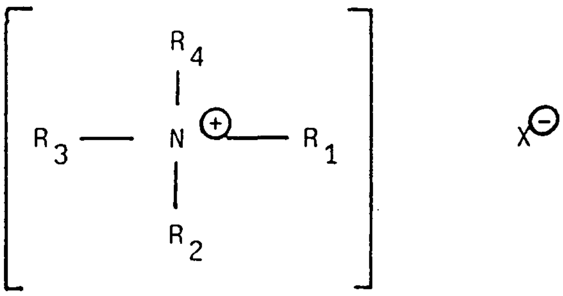

(I)

erhalten worden sind, in der zumindest einer der Reste $R_1$ bis $R_4$ ein Kohlenwasserstoffrest mit ≧ 8 C-Atomen ist, während die übrigen Reste R gleich oder verschieden sein können und für Niederalkyl, insbesondere für $CH_3$- oder ggf. $C_6H_5$-$CH_2$- stehen und X ein verträgliches Anion, insbesondere Chlorid oder Bromid bedeutet.

**4.** Verfahren nach Anspruch 3,
**dadurch gekennzeichnet,**
daß man mit Hilfe von Verbindungen der Formel (I) permeabilisierte Zellen verwendet, bei denen $R_1$ und ggf. $R_2$ ein Alkylrest mit 10 bis 16 C-Atomen ist und die übrigen Reste R $CH_3$-Gruppen bedeuten, von denen eine einen Phenylrest tragen kann und X für Chlorid oder Bromid steht.

**5.** Verfahren nach einem der vorangehenden Ansprüche,
**dadurch gekennzeichnet,**
daß man mit 0,1 bis 0,3 %iger Tensidlösung bei Zimmertemperatur 1 bis 10 Minuten lang permeabilisierte und durch Auswaschen von überschüssigem Tensid befreite Zellen verwendet.

**6.** Verfahren nach einem der vorangehenden Ansprüche,
**dadurch gekennzeichnet,**
daß man die Stoffumwandlung unter Einsatz von Zellkonzentrationen permeabilisierter Zellen von 20 bis 80 g/l (Zelltrockenmasse) durchführt.

**7.** Permeabilisierte Zellmasse von Zymomonas mobilis, dadurch gekennzeichnet daß sie durch Permeabilisierungsbehandlung mit Kationtensid hergestellt wurde.

**8.** Permeabilisierte Zellmasse nach Anspruch 7, immobilisiert auf einem Trägermaterial.

**Claims**

**1.** Process for obtaining sorbitol and gluconic acid or gluconate starting from aqueous glucose/fructose mixtures by transformation in the presence of permeabilized cells of Zymomonas mobilis, characterized in that cells permeabilized by treatment with an at least 0.1% strength cationic surfactant solution are used.

**2.** Process according to Claim 1, characterized in that permeabilized cells which have been obtained by treatment with a long-chain quaternary alkylammonium compound are used.

**3.** Process according to Claim 2, characterized in that permeabilized cells which have been obtained by treatment with a compound of the formula (I)

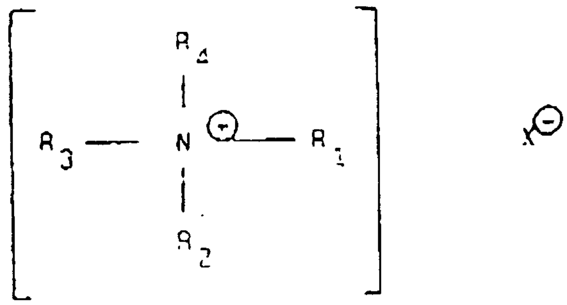

(I)

in which at least one of the radicals $R_1$ to $R_4$ is a hydrocarbon radical with ≧ 8 C atoms, while the other radicals R can be identical or different and represent lower alkyl, in particular $CH_3$- or, where appropriate, $C_6H_5$-$CH_2$-, and X is a tolerated anion, in particular chloride or bromide, are used.

**4.** Process according to Claim 3, characterized in that cells permeabilized with the aid of compounds of the formula (I) where $R_1$ and, where appropriate, $R_2$ is an alkyl radical with 10 to 16 C atoms, and the other radicals R denote $CH_3$ groups, of which one can carry a phenyl radical, and X represents chloride or bromide, are used.

**5.** Process according to any of the preceding claims, characterized in that cells which have been permeabilized with 0.1 to 0.3% strength surfactant solution at room temperature for 1 to 10 minutes and from which excess surfactant has been removed by washing out are used.

**6.** Process according to any of the preceding claims, characterized in that the transformation is carried out using cell concentrations of permeabilized cells of from 20 to 80 g/l (cell dry matter).

**7.** Permeabilized biomass of Zymomonas mobilis, characterized in that it has been prepared by permeabilizing treatment with cationic surfactant.

**8.** Permeabilized biomass according to Claim 7, immobilized on a carrier material.

**Revendications**

**1.** Procédé d'obtention de sorbitol et d'acide gluconique ou de gluconate à partir de mélanges aqueux de glucose et de fructose , par transformation de ces substances en présence de cellules de *Zymomonas mobilis* rendues perméables, caractérisé en ce qu'on utilise des cellules rendues perméables avec une solution contenant au moins 0,1 % d'agent tensio-actif cationique.

**2.** Procédé selon la revendication 1, caractérisé en ce qu'on utilise des cellules rendues perméables, qu'on a obtenues grâce à un traitement avec un composé d'alkylammonium quaternaire à chaîne longue.

**3.** Procédé selon la revendication 2, caractérisé en ce qu'on utilise des cellules rendues perméables, qu'on a obtenues grâce à un traitement avec un composé de formule (I) :

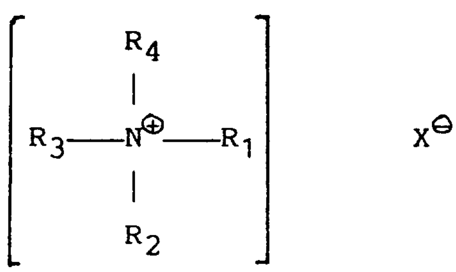

(I)

dans laquelle au moins un des restes $R_1$ à $R_4$ est un reste d'hydrocarbure contenant 8 atomes de

carbone ou plus, tandis que les autres restes R peuvent être identiques ou différents et représentent un groupe alkyle inférieur, en particulier $CH_3$- ou éventuellement $C_6H_5$-$CH_2$-, et X signifie un anion compatible, en particulier chlorure ou bromure.

**4.** Procédé selon la revendication 3, caractérisé en ce qu'on utilise des cellules rendues perméables à l'aide de composés de formule (I), dans lesquels $R_1$ et éventuellement $R_2$ signifient un reste alkyle ayant de 10 à 16 atomes de carbone, et les autres restes R signifient des groupes $CH_3$, dont l'un d'entre eux peut porter un reste phényle, et X représente un chlorure ou un bromure.

**5.** Procédé selon l'une des revendications précédentes, caractérisé en ce qu'on utilise des cellules rendues perméables avec une solution contenant 0,1 à 0,3 % d'un agent tensio-actif, à la température ambiante, pendant 1 à 10 minutes, et débarrassées de l'agent tensio-actif en excès par lavage.

**6.** Procédé selon l'une des revendications précédentes, caractérisé en ce qu'on effectue la transformation des substances en utilisant des concentrations de cellules rendues perméables comprises entre 20 et 80 g/l (masse de cellules à l'état sec).

**7.** Masse de cellules de *Zymomonas mobilis* rendues perméables, caractérisée en ce qu'on l'a préparé grâce à un traitement de perméabilisation avec un agent tensio-actif.

**8.** Masse de cellules rendues perméables selon la revendication 7, immobilisée sur un matériau de support.